# EUROPEAN PATENT APPLICATION

(11) **EP 3 607 956 A1**
(43) Date of publication of application: **12.02.2020**
(21) Application number: 18776433.7
(22) Date of filing: 02.04.2018
(51) Int. Cl.: A61K 33/04, A61K 35/66, A61P 31/00, A61P 31/10, B82Y 5/00

(54) **BIOPRODUCT BASED ON SELENIUM NANOPARTICLES IN A HONEY MATRIX FOR THE TREATMENT OF COMPLEX INJURIES AND DERMATOLOGICAL INFECTIONS**

(30) Priority: 30.03.2017 CL 2017766
(71) Applicant: Arroyo Pérez, Patricia Janette, Concepcion (CL); Vergara Soto, Marcelo Alexis, San Pedro De La Paz (CL)
(72) Inventor: ARROYO PÉREZ, Patricia Janette, San Pedro De La Paz (CL)
(74) Representative: Carvajal y Urquijo, Isabel
(86) International application number: PCT/CL2018/050017
(87) International publication number: WO 2018/176168

(57) **Abstract**

The invention relates to an antiseptic dermatological bioproduct for treating complex injuries and controlling fungal and dermatophyte skin infections, the production thereof comprising selenium nanoparticles, in which said selenium nanoparticle is produced in the natural honey matrix. Said bioproduct is applied in the form of a gel, cream or dressing.

## Description

### FIELD OF THE INVENTION

The present invention will be used in the health area for the treatment of complex injuries and dermatological infections caused by microorganisms or by chronic diseases.

### STATE OF THE ART

Fungal infections, superficial mycoses are, among skin diseases, a prevalent dermatological pathologies and are produced by two large groups of fungi: yeasts and dermatophytes. The first occur due to an alteration of the microbiota that leads to a fungi proliferation and the second ones are skin exogenous infections.

The most important fungi in dermatology are *Candida* spp. and *Malasezzia* spp. Candidiasis is a problem that manifests itself at a superficial level or in very specific areas, and is usually topically and/or systemically attacked, the latter by intake of pharmaceutical products.

Anti-Candida products belong to synthetic products such as azoles, ketoconazole, fluconazole, clotrimazole, etc. none of them having natural features.

Nanomedicine is one of the most promising aspects within the potential new technological medical advances. At present, this area has a great development in biomedical applications, especially in pharmaceutical technology such as controlled drug release systems. These systems are characterized in terms of their properties of vectorizing drugs, peptides, genes, etc. for targeting specific cells or tissues.

Nanomaterials are those materials that, at least in one dimension, are smaller than 100 nm and comprise basic structural units such as grains, fibers, particles or other components. These nanomaterials favor a greater contact surface resulting in superior physicochemical (mechanical, electrical, magnetic, optical, catalytic ...) properties and favoring the development of applications in fields as diverse as biomedicine, electronics, information technology, etc.

Nanoparticles synthesis has undergone a great advance in recent decades due to the high number of applications in fields such as cosmetics, industry, and especially in medicine.

Nanoparticles properties depend on the optical, magnetic and electrical size of particles, which have shown to be promising candidates for *in vivo* applications such as detection, catalysis, therapy and target cells. Lately, the use of drugs using nanotechnology or the detection of diseases with nanoparticles has generated a great excitement because this is a mechanism that is not painful and does not breakdown in the intestinal tract as most proteins and polypeptides do, which for the common people represents a good health alternative. Numerous investigations have shown that transporting nanoparticles can improve the therapeutic agent stability against enzymatic degradation, achieving results and directly reaching the targets (specific tissues or cells)

There are different ways to produce nanoparticles, which are classified into two large groups, the top-down method, which is the continuous breaking of a material and the bottom-up method that consists of the construction of nanomaterials from their constituents. On the other hand, nanoparticle production can be classified into physical, chemical or biological synthesis methods. Among these are the following methods:
- Physical methods: inert-gas condensation, electric arc discharge, laser cutting, pyrolysis and spray pyrolysis.
- Chemical methods: metal reduction, solvothermal synthesis, photochemical synthesis, electrochemical synthesis, thermolysis routes, sonochemical routes, micelles and microemulsions, liquid-liquid interfaces.
- Biological methods: microorganisms are the reactors and carry out nanoparticles synthesis.
- Hybrid methods: it is a mixture of the previous methods; such as laser synthesis, Postsynthetic Size-Selective Processing and Solvated Metal Atom Dispersion (SMAD).

Nanoparticle formation in the vast majority of the synthetic methods and routes depends on the nucleation that takes place at the time of their synthesis and the stability given to the nanometric particle when formed. This is why chemical methods are the most widely used due to their potential to adequately control nanoparticle size, size distribution and shape. Traditional chemical methods use reducing and stabilizing agents during synthesis that are toxic and contaminant to some extent. The trend for years has focused on developing synthesis methods under the concept of "Green Chemistry", which is the one that uses nontoxic and environmentally friendly chemicals.

Metal nanoparticle antimicrobial activity is known to be a function of the surface area contacting the microorganisms. Transition from microparticles to nanoparticles involves an increase in the contact area/volume ratio (aspect ratio), wherein the contact area increases dramatically allowing a greater number of interactions with organic, inorganic molecules and bacteria.

There are reports of antimicrobial activity of different metal nanoparticles such as gold, silver, copper, titanium and zinc. Despite this, the bactericidal mechanisms of these have not yet been fully understood.

In relation to the state of the industrial property, the following documents related to the technology subject matter of this application were found.

US Patent US2013/0273020 describes a formulation comprising honey, or an active fraction thereof, plus lactoferrin. This synergistic formulation inhibits yeast growth more than its components separately.

On the other hand, the Chinese patent CN103704552 describes a nutritional composition comprising honey, pollen, propolis, royal jelly, extracts from different plants used in Chinese medicine and traces of Selenium. This composition allows to form an edible paste beneficial for health.

In addition, there are documents such as articles and/or publications related to the technology wanted to be protected, which refer to antifungal and antibacterial properties of honey, in addition to other properties, although these documents relate to honey properties, these technologies are totally different from the subject matter for which protection is herein wanted.

### BRIEF DESCRIPTION OF THE FIGURES

**Figure 1**: This figure corresponds to the visualization of nanoparticles of Selenium in a honey matrix of about 33 nm of diameter.
**Figure 2**: Comparative graph between pharmaceutical products currently available in the market, nanoparticles of Selenium in a honey matrix and other natural products. MIC, minimum inhibitory concentration on *Candida albicans* by: 1, Clotrimazole; 2, Terbinafine; 3, Miconazole Nitrate; 4, commercial phytopharmaceutical Kolorex; 5, Natural active principle of Chilean plant + Ulmo honey; 6, Honey; 7, Selenium Nanoparticle Bioproduct in a honey matrix; 8, Manuka; 9, Tea; 10, thuja and tea; 11, Calendula and Equisetum.
**Figure 3**: Antifungal activity of nanoparticles against *Candida albicans*,
   **3A** this figure shows the inhibitory effect on *Candida* spp. strain growth by Silver Nanoparticles (25); Copper Nanoparticles (26); Commercial Anti-Candida Product Kolorex (27).
   **3B** this figure shows the inhibitory effect on *Candida* spp. strain growth by Selenium Nanoparticle Bioproduct in a honey matrix (MCSII); Commercial anti-Candida product Kolorex (two tablets) (MK30).

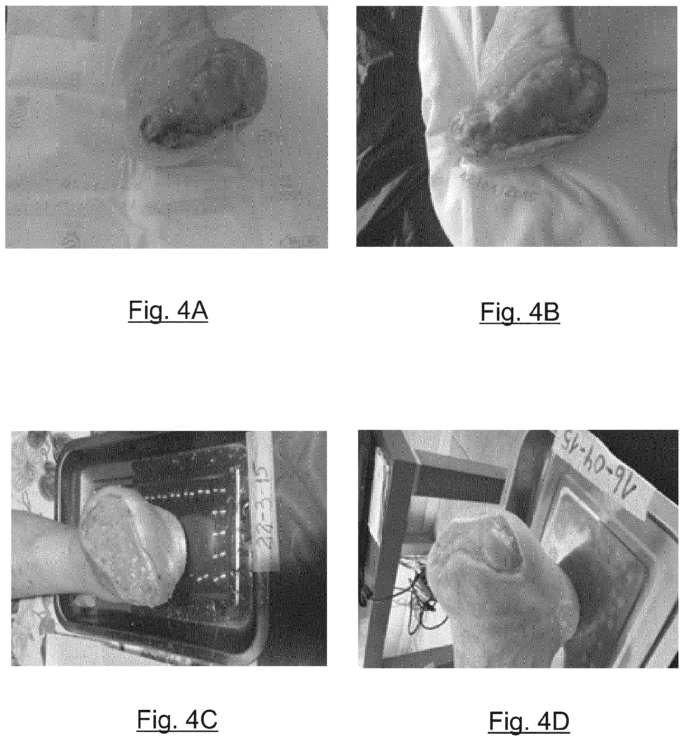

Figure 4: This figure shows the evolution of an injury after applying the honey product with Selenium nanoparticles
   4A, Initial injury, without applying the product.
   4B, Injury after 3 days of product application
   4C, Injury at 2 months, applying product 2 times per week.
   4D, Injury evolution, tissue reconstitution is observed with Selenium nanoparticle treatment in Ulmo honey.

### DESCRIPTION OF THE INVENTION

The present technology describes a bioproduct, corresponding to an antiseptic to be used in the control of fungi and dermatophytes in the skin and in the treatment of injuries, the elaboration comprises: 1) an antiseptic matrix capable of providing antimicrobial activity, comprising about 70% of honey and 2) Selenium nanoparticles in the form of Se°; wherein said particles are stabilized by the natural honey matrix.

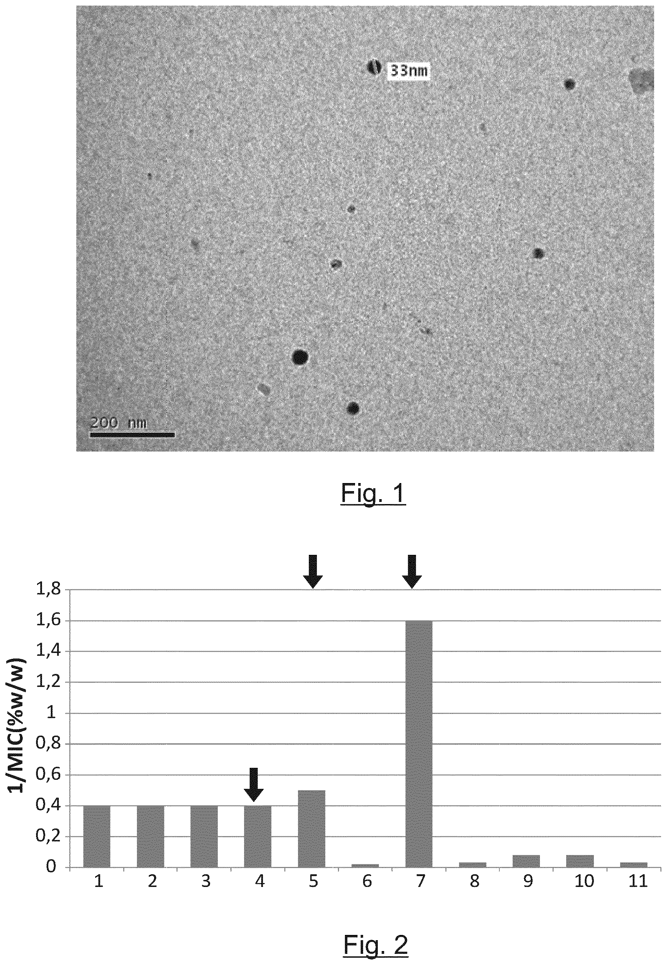

The antimicrobial composition with Selenium nanoparticles, comprises a size of 30 to 60 nm, preferably 33 nm of average diameter (**Figure No. 1**); its weight is about 0.01% up to 15% weight stabilized within the honey matrix containing between 20% to 80% water.

The bioproduct is primarily used as an antiseptic formulation for dermatological treatment including complex injuries, such as diabetic foot; and for the treatment of fungal, yeasts and bacterial infections, preferably for treating infections by *Candida* spp., *Staphylococus Aureus*, among others, in humans and animals.

This proposal consists of enhancing the intrinsic properties provided by the Selenium nanoparticles in a honey matrix. The bioproduct obtained has increased antifungal, antibacterial and tissue regenerating properties, which is given by the formation of Selenium nanoparticles from the sodium selenite salts in the honey matrix through a green synthesis.

An inverse relationship between the nanoparticle size and its antimicrobial activity has been demonstrated, where nanoparticles having a size range of 1-10 nm have been shown to have the highest antibacterial activity.

The bioproduct was subjected to various test, including comparison studies (**Figure 2**) between: natural products, synthesis products and nanoparticles in Selenium honey matrix, for controlling *Candida albicans,* a pathogen that colonizes patients having a compromised immune system.

The graph shows that the inhibitory activity in *Candida* spp. of the formulated natural product, Selenium nanoparticles in a honey matrix, is at least 4 times higher than market products (**7**, **figure No. 2**).

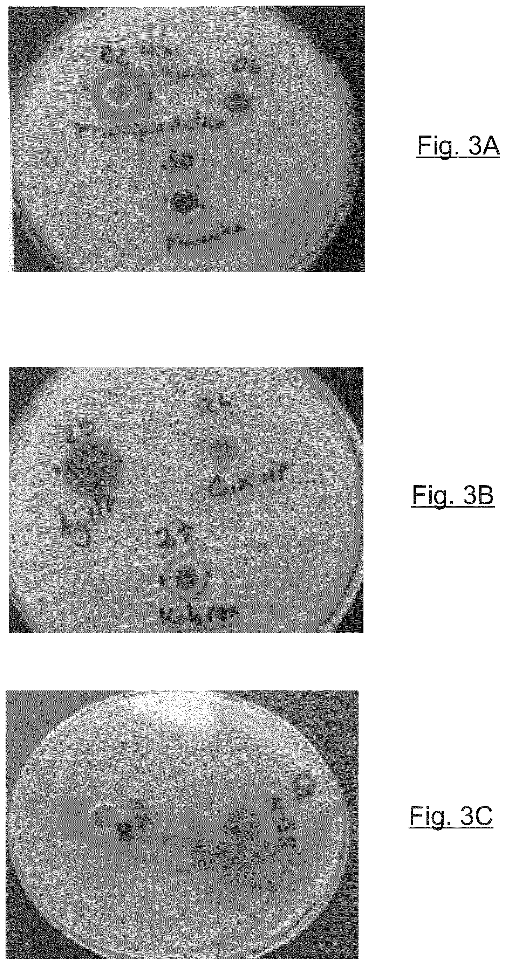

Study results are also seen in **Figure No. 3,** where the inhibition halo of each of the tested compounds is observed; **Figure 3A** shows *Candida* spp. growth inhibition halos by honey and an internationally marketed natural product used to control *Candida*, which is Kolorex (02); honey (06) and Manuka honey (30). **Figure 3B** shows inhibition halos by Silver nanoparticles (25); Copper nanoparticles (26); Commercial Anti-Candida Product Kolorex (27). And finally **Figure 3C** shows inhibition halos by the Selenium nanoparticle bioproduct in a honey matrix (MCS11) and anti-Candida Commercial Product Kolorex (MK30) (two tablets), the bioproduct shows an inhibition halo of at least twice of the inhibition halo produced by copper, silver nanoparticles and commercial product Kolorex, seen above, as shown by **Figure 3C**, being greater or different for other microorganisms.

A clinical trial was carry out to verify the efficacy of this bioproduct in humans, the product was applied directly to the injury of an 80-year-old patient with severe obstructive arterial disease, diabetes mellitus, hypertension and with previous amputation of the lower limb. The injury was an open transmetatarsal amputation since there was prior staphylococcal colonization of the skin. The product was applied and then covered with non-synthetic gauze and common dressing. The treatment was carried out by applying the product 3 times per week; the product was left for 2 days in the most acute phase, i.e., more exudative. Thanks to a good evolution of the injury, the application of the product was subsequently distanced to 2 times per week and the use of low intensity compression bandage was added.

**Figure No. 4** shows the evolution of the surgery injury, as the product was applied; **Figure 4A** shows the initial infected injury; **Figure 4B** shows the injury three days after the application of the product, showing a cleaner injury compared to how the patient arrived.

**Figure 4C** shows the injury after two months of treatment and **Figure 4D** clearly shows the tissue reconstitution in the injury. This demonstrates that this product helps, in complex injuries, to infection recovery and tissue reconstitution.

The dosage form of this bioproduct is gel; creams; dressings for the treatment of injuries, preferably complex injuries; fungal and/or bacterial infections.

### APPLICABILITY EXAMPLES

### Example No. 1: Product Formation Methodology

The formulation of the bioproduct based on honey and Selenium nanoparticles was carried out by a process that first required generating a sodium selenite solution (1 M), which was mixed with honey to obtain a very viscous solution at a concentration of micromolar Selenium in honey, which finally contained 25% water. Honey fluidity was increased to be mixed by first raising and keeping the temperature at 45 °C. Selenium nanoparticles starting to form by raising and keeping the temperature of the mixture at 60 °C, this was made on a rotary evaporator. Formation of Selenium nanoparticles was achieved by the great reducing power of honey thanks to the presence of monosaccharides, mainly glucose, and an acid pH of about 4.0 to 4.5. Sodium selenite under these conditions slowly begins to reduce to red Se°, forming Selenium nanoparticles of about 20 nm to 33 nm. Starting from this viscous solution, a lower viscosity honey solution can be formed or 940 carbomer can be used to form a less viscous gel or form a solid dressing in an alginate matrix, incorporating the honey in layers.

### Example No. 2: Studies conducted with Candida albicans

Comparison studies were conducted between: natural products, synthetic products and nanoparticles of a metal for controlling *Candida albicans*, a pathogen that colonizes patients having a compromised immune system.

*Candida albicans* is grown in Sabouraud medium at 37 °C for 24 to 48 hours with different products to measure the minimum inhibitory concentration (MIC). 0.025 mg/ml Clotrimazole; 0.025 mg/ml Terbinafine; 0.025 mg/ml Miconazole nitrate; Commercial phytopharmaceutical Kolorex (2 tablets); Natural product of native plant + honey; Honey; 0.00625 mg/ml Selenium Nanoparticle Bioproduct in a honey matrix; thuja and tea; Calendula and Equisetum were used.

This study shows that the growth inhibitory effect on *Candida* spp. of the Selenium Bioproduct in a honey matrix is significantly greater than that of the components both drugs and natural products available in the market.

The results of this study are also shown by Figures 3A, B and C, where the inhibition halo of each of the compounds tested is observed. This test shows that the Selenium Bioproduct in a honey matrix produces an inhibition halo of 2 and up to 3 times greater than that of conventional products such as silver and copper nanoparticles, an also of the commercial anti-Candida product Kolorex.

### Example No. 3: Test of the Nanoparticle Bioproduct in a honey matrix

The product was directly applied on the injury with a sterile instrument, after cleaning with physiological serum, then covered with non-synthetic gauze and common dressing. The product is left for 2 days in the most acute phase, i.e., more exudative. Dressing was made 3 times a week, due to a good evolution of the injury it was then distanced to 2 times per week and use of low intensity compression bandage was added. It was applied to an 80-year-old patient with severe obstructive arterial disease, diabetes mellitus, hypertension with previous amputation of the other lower limb. The injury was an open transmetatarsal amputation since there was prior staphylococcus colonization of the skin. 1 g Vitamin C and zinc was added to the diabetic regimen. Another indications were elevated leg and some exercises to stimulate circulation.

## Claims

1. A dermatological antiseptic bioproduct for the treatment of complex injuries, control of fungal infections and dermatophytes in skin **CHARACTERIZED in that** its elaboration comprises: 1) an antiseptic matrix having antimicrobial activity, comprising about 70% honey, and 2) Selenium nanoparticles; wherein said Selenium nanoparticle is produced in the natural honey matrix.

2. A dermatological bioproduct according to claim 1, **CHARACTERIZED in that** the Selenium nanoparticles comprise a particle size from 30 to 60 nm, preferably 33 nm of average diameter.

3. A dermatological bioproduct according to claim 1, **CHARACTERIZED in that** the metal nanoparticles comprise at least 0.01% up to 15% weight stabilized within the honey matrix comprising from 20% to 80% water.

4. A dermatological bioproduct according to claim 1, **CHARACTERIZED in that** the inhibitory activity on *Candida* spp. by the bioproduct, Selenium nanoparticles in honey matrix, is at least 4 times higher than market products.

5. A dermatological bioproduct according to claim 1, **CHARACTERIZED in that** the Selenium nanoparticle bioproduct in honey matrix produces an inhibition of at least twice of the inhibition halo produced by copper, silver nanoparticles and commercial product Kolorex, in a culture of *Candida* spp.

6. A dermatological bioproduct according to claim 1, **CHARACTERIZED in that** said bioproduct comprises tissue regenerating properties.

7. A dermatological bioproduct according to claim 1, **CHARACTERIZED in that** the antiseptic formulation is mainly used for the dermatological treatment of fungi, yeasts and bacteria, preferably for treating infections by *Candida* spp. in humans and animals.

8. A dermatological bioproduct according to claim 1, **CHARACTERIZED in that** the dosage form of said bioproduct is gel, creams and/or dressings.
